(19) Europäisches Patentamt
European Patent Office
Office européen des brevets

(11) Veröffentlichungsnummer: **0 324 875 A1**

(12) **EUROPÄISCHE PATENTANMELDUNG**

(21) Anmeldenummer: **88100806.4**

(22) Anmeldetag: **20.01.88**

(51) Int. Cl.[4]: **A61K 31/535 , //(A61K31/535, 31:13)**

(43) Veröffentlichungstag der Anmeldung: **26.07.89 Patentblatt 89/30**

(84) Benannte Vertragsstaaten: **AT BE CH DE ES FR GB IT LI**

(71) Anmelder: **S O "PHARMACHIM"**
**Iliensko Chaussée 16**
**Sofia(BG)**

(72) Erfinder: **Galabov, Angel Simeonov, Dr.**
**7b, Krestyu Sarafov Strasse**
**Sofia(BG)**
Erfinder: **Velitchkova, Emilia Hristova, Dr.**
**28-A, Dospat Strasse**
**Sofia(BG)**
Erfinder: **Uzunov, Sergey Todorov**
**9, Sim. Slavev Strasse**
**Sofia(BG)**
Erfinder: **Dantchev, Damyan Kanev**
**173, Pozitano Strasse, Block 80**
**Sofia(BG)**
Erfinder: **Sidjakova, Dorotea Ivanova**
**Block 8-B, Mlad. Prohod Strasse**
**Sofia(BG)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Antivirus-Mittel.**

(57) Das erfindungsgemäße Antivirus-Mittel wird für die Chemieprophylaxe und Chemietherapeutik von Grippe mit einer höheren und einer ein breiteres Spektrum aufweisenden antivirellen Wirksamkeit eingesetzt.

Das Mittel enthält die Verbindung (I)

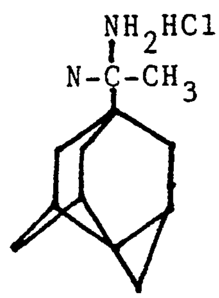

N-Methyl-1-adamantanmethylamin-Hydrochlorid

in Kombination mit der Verbindung (II)

EP 0 324 875 A1

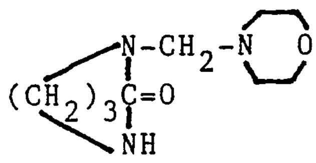

pyrimidinon

1-Morpholinomethyl-tetrahydro-2/1H/-~~Pyramidon~~

# ANTIVIRUS-MITTEL

Die Erfindung betrifft ein Antivirus-Mittel, das zur Behandlung und Prophylaxe von Virus-Erkrankungen in der Humanmedizin Anwendung finden wird.

Als Arzneimittel mit Antivirus-Wirksamkeit und bekannt N-Methylisatin-β-thiosemicarbazon, 5-Jod-2′-desoxyuridin, 5-Trifluormethyl-2′-desoxyuridin, Adenin-Arabinosid, Zytosin-Arabinosid, 5-Ethyl-2′-desoxyuridin, N-Dimethylaminoethoxyacetyl-Adamantanamin-Hydrochlorid, 9-/2-Hydroxyethoxymethyl/Guanin/Acycloguanosin und andere, die für die Behandlung von Herpes Simplex, Herpes Zoster, Variola und Komplikationen nach einer Impfung gegen Variola, Windpocken (Varizelle) (Sidwell, R.W., and J.T. Witkowski, Burger's Medicinal Chemistry (M.E. Wolf, ed.), Part II. Wilies & Sons Inc., New York, pp. 543-593 1979; Galasso, G.J. Amer. Soc. Microbiol. News 45, 353-358 1979; Antiviral Agents and Viral Diseases of Man (G.J. Galasso, T.C. Meridan and R.A. Buchanan, eds.), Raven Press, New York 1979; Galabov, A.S., Sb. "Savremenni Problemi na Infektiosnata Pathologia, H.6, Medizina i Fiskultura, Sofia, S. 51-81 1981) benutzt werden.

Unter den antivirellen Mitteln, die zur Behandlung und Prophylaxe von Grippe beim Menschen angewandt werden, sind Adamantanamin-Hydrochlorid, N-Methyl-1-adamantanmethylamin-Hydrochlorid/Rimantadin, Remantadin/ und andere Derivate des Adamantanamins, 1-Þ-D-Ribofuranosil-1,2,4-Triazol-3-Karbonsäureamid /Ribavirin, Virazol/ (Sidwell, R.W., and J.T. Witkowski, Burger's Medicinal Chemistry (M.E.Wolf, ed.), Part II. Wilies & Sons Inc., New York, pp. 543-593 (1979); Galasso, G.J., Amer.Soc.Microbiol. News 45, 353-358 (1979); Antiviral Agents and Viral Diseases of Man (G.J. Galasso, T.C. Meridan and R.A. Buchanan, eds.), Raven Press, New York (1979); Galabov, A.S., Sb. "Savremenni Problemi na Infektiosnata Pathologia", H.6, Medizina i Fiskultura, Sofia, S.51-81 (1981; Eldnikov, D.M., A.P. Kazanzev i P.D.Starschov - "Therapija Virusnich Bolesnej", Medizina, Leningrad (1979)). Mit besonders hoher Effektivität zeichnet sich das Rimantadin aus (Galasso, G.J., Amer. Soc.Micorobiol.News 45, 353-358 (1979); Galabov, A.S., Sb. "Savremenni Problemi na Infektiosnata Pathologia, H.6, Medizina i Fiskultura, Sofia, S. 51 - 81 (1981); Eldnikov, D.M., A.P.Kazanzev i P.D. Starschov - "Therapija Virusnich Bolesnej", Medizinia, Leningrad (1979)).

Die Anwendung von Rimantadin und Adamantanamin-Hydrochlorid als Antigrippe-Mittel birgt zwei ernste Probleme:

- Das Spektrum der gegen diese Stoffe empfindlichen Grippe-Viren ist nicht vollständig - es schließt nicht die Stämme des Grippe-Virus B ein.
- Es besteht eine reelle Gefahr der Entstehung von widerstandsfähigen(resistenten) gegenüber dem Adamantanamin und Rimantadin Mutanten des Grippe-Virus A bei der breiten Anwendung der Präparate zur Prophylaxe und Behandlung der Grippe. Dies wurde bei Versuchen eindeutig bewiesen.

In Bezug auf das Adamantanamin-Hydrochlorid ist noch zu erwähnen, daß dieses sich durch einen verhältnismäßig niedrigen Wert der therapeutischen Breite (Index der selektiven Wirksamkeit) auszeichnet.

Was das Rivavirin angeht samt einigen widersprüchlichen Daten über seine klinische Wirksamkeit bei Grippe-Infektionen, ist bekannt, daß keine volle Garantie für seine Unschädlichkeit besteht (Galasso, G.J., Amer.Soc. Microbiol.News 45, 353-358 (1979); Galabov, A.S., Sb. "Savremenni Problemi na Infektiosnata Pathologia", H.6, Medizina i Fiskultura, Sofia, S. 51-81 (1981)), was zur Zeit ein wichtiges Hindernis für seine Anwendung als Antigrippe-Mittel (peroral) ist.

Aufgabe der Erfindung war es nun, ein Mittel für eine Chemieprophylaxe und Chemietherapeutik von Grippe mit einer höheren und einer ein breiteres Spektrum aufweisenden antivirellen Wirksamkeit bereit zu stellen.

Diese Aufgabe wird gelöst, indem die Verbindung (I)

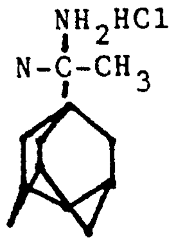

N-Methyl-1-adamantanmethylamin-Hydrochlorid

in Kombination mit der Verbindung (II) eingesetzt wird

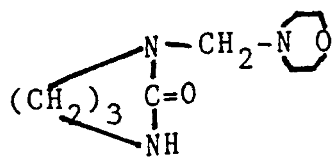

1-Morpholinomethyl-tetrahydro-2/1H/-pyrimidinon

Verbindung (I), Rimantadin ist ein klinisch erprobtes Mittel zur Chemieprophylaxe und Chemietherapeutik von Grippe-Infektionen beim Menschen, mit einem Verursacher - Stämmen des Grippe-Virus A (Sidwell, R.W., and J.T. Witkowski, Burger's Medicinal Chemistry (M.E. Wolf, ed.), Part II. Wilies & Sons Inc., New York, pp 543-593 (1979); Galasso, G.J., Amer.Soc. Microbiol.News 45, 353-358 (1979); Antiviral Agents and Viral Diseases of Man (G.J.Galasso, T.C. Meridan and R.A. Buchanan, eds), Raven Press, New York (1979); Galabov, A.S., Sb. "Savremenni Problemi na Infektiosnata Pathologia", H.6, Medizina i Fiskultura, Sofia, S. 51-81 (1981)); Eldnikov, D.M., A.P. Kazanzev i P.D. Starschov - "Therapija Virusnich Bolesnej", Medizina, Leningrad (1979)), und Verbindung (II) ist ein Stoff mit einer bewiesenen antivirellen Wirksamkeit bei Infektionen mit Grippe-Viren A und B und mit Togaviren [Urheberschein-Nr. 38396/1978; Urheberschein-Nr. 53809/1981].

Die Wirksamkeit des erfindungsgemäßen antivirellen Mittels - Kombination von zwei antivirellen Stoffen - wird wie folgt festgestellt:

- Agar-Diffusions-Plaque-Inhibitions-Test von Rada und Závada;
- Plaque-Reduktions (Plaque-Inhibitions) Verfahren von Hermann - Simonoff;
- Mehrcyclen-Test;
- Eincyclus-Test (Einstellen eines Einschritt-Virus-Fortpflanzungscyclus).

Das Agar-Diffusionsverfahren [Rada, B., J.Závada, Neoplasma 9, 57-63 1962)] sum Testen der kombinierten Wirksamkeit wird auf einschichtigen Zellkulturen vom embryonalen Hühnerfibroblastemen (EHF) in Petrischalen mit einem Durchmesser von 90 mm durchgeführt. Die letzteren werden mit dem Virus FPV/A/H7 N7/ Stamm Weibridge / in einer Dosis inokuliert, die halbkonfluierende Plaques nach 72-stündiger Inkubationsdauer bei einer Temperatur von 37° C ergibt. Es wird eine Agar-Deckschicht aufgelegt, in der Rimantadin in unterschiedlichen für die Zellen nicht toxischen Konzentrationen enthalten ist. In der Agarschicht werden kleine Glaszylinder fixiert, in denen ein Tetrahydropyrimidinon-Derivat (II) mit einer Kodebezeichnung DD-13 (Wasserlösung) eingetropft wird. Es werden Kontrollen der Virus-Dosis, der selbständigen Wirksamkeit des Rimantadins und des DD-13, sowie auch derer Zytotoxizität angelegt. Die antivirelle Wirksamkeit des DD-13 wird auf Grund des Ausmaßes (Durchmesser in mm) der Inhibitionszone (die keine Plaques enthält oder Plaques mit vermindertem Durchmesser enthält) und der zytotoxischen Zone abgerechnet.

Beim Einstellen des Plaque-Reduktionstestes (Hermann, E.C., Proc. Soc.Exp.Biol.Med. 107, 142 - 145 (1961); Simonoff, P., App. Microbiol., 9, 66-72 (1961) wird die Zellenmonoschicht (Zellen EHF) in einer Petrischale mit einem Durchmesser von 70 mm mit 100 bis 1000 Plaque-bildenden Einheiten (PBE) Virus FPV inokuliert. Die Verbindungen werden in geeigneten Konzentrationen in die Agarschicht eingeschlossen. Die Kulturen werden während 72 Stunden bei einer Temperatur von 37° C inkubiert. Die Anzahl der Plaque-bildenden Einheiten wird ausgerechnet und das Inhibitionsprozent mit einem Vergleich der Plaque-Anzahl in der Kontrolle (nicht enthaltend beide Verbindungen in der Agarschicht) berechnet. Die Einschätzung für den Charakter der kombinierten Wirksamkeit erfolgt nach einer Gleichung, die zur Prüfung der Wirkung von Inhibitoren der Enzym-Reaktionen und in der Pharmakologie [Uebb, L., "Inhibitori Fermentow i Metabolisma (obschtie Prinzipi Tormojenija)", Mir, Moskau, S. 484 - 486 (1966)] angewandt wird:

$$i_{1,2} = i_1 + i_2 - i_1 i_2$$

$$i_1 = \frac{\text{\% Inhibition der Verbindung ( I ) (selbständiger)}}{100}$$

$$i_2 = \frac{\text{\% Inhibition der Verbindung (II ) (selbständiger)}}{100}$$

$$i_{1,2} = \frac{\text{\% Inhibition bei der Kombination von (I) + (II)}}{100}$$

bei einem additiven Effekt $i_{1,2} = i_1 + i_2 - i_1 i_2$
bei einem Antagonismus $i_{1,2} < i_1 + i_2 - i_1 i_2$
bei einem Synergismus $i_{1,2} > i_1 + i_2 - i_1 i_2$

Bei den Mehrcyclen- und Eincyclustests werden einschichtige Zellkulturen einer embryonalen Kalbsniere verwendet, welche mit Grippe-Viren A/H3N2/Texas/77 und B/Lii/MOI = 0.01-0.5 und 1-10 entsprechend infiziert werden. Die Ausbeute an infektiösem und hämaglutinierendem Virus wird in der 24sten Stunde (bei einem Mehrcyclentest) und entsprechend in der zwölften bis fünfzehnten Stunde (bei einem Eincyclustest) ausgerechnet. Der infektiöse Titer wird $EID_{50}$/ml bestimmt. Dieselben Versuche werden auch mit Virus FPV (Stamm Weibridge) in Zellen EHF(bei Ausrechnung des infektiösen Virus Plaque-bildenden Einheiten und des hämaglutinierenden Virus) durchgeführt. Die Wirksamkeit der Verbindungen wird im Bezug auf die Kontrolle (ohne Inhibitoren) ausgerechnet.

Beim Verfolgen der Wirkung des DD-13 auf die Entwicklung einer Resistenz gegen Rimantadin werden aufeinanderfolgende Passagen der Kultivierung des Virus FPV in Zellen EHF (in Reagensgläsern) in Anwesenheit nur von Rimantadin in Konzentrationen im Bereich 25 - 150 mkg/ml (d.h. unter oder ein wenig über der für die Zellen maximal verträglichen Konzentration) durchgeführt und parallel damit Passagen des Virus in denselben Konzentrationen des Rimantadins + 60 mkg/ml DD-13. Nach jeder dritten Passage bei einem Eincyclustest wird die Empfindlichkeit von jedem Zweig-Virus zum Rimantadin durch Bestimmung der Ausbeute des infektiösen Virus (in Plaque-bildenden Einheiten/ml) geprüft.

Die antivirelle Wirksamkeit der Kombination wird auch bei der Prüfung in vivo - bei experimentellen Infektionen bei weißen Mäusen mit Grippe-Viren A/H3N2/Aichi, A/H1N1/Porto Riko 8, B/Lii festgestellt. Es werden weiße Mäuse von der Linie H oder Suis mit einem Gewicht von 10 g intranasal angesteckt. Die Verbindungen werden peroral in Form von Wasserlösungen (hergestellt ex tempore) angewandt. Über die antivirelle Wirksamkeit wird nach der Dynamik des kumulativen Prozentes der Letalität während der Beobachtungsperiode von 14 Tagen (im Vergleich zur Placebogruppe) beurteilt. Es werden der Protektionskoeffizient (PK) und der Schutzindex (SI) sowie auch die durchschnittliche Zeit des Überlebens (DZÜ) berechnet.

Im Agar-Diffusionstest überschreitet die antivirelle Wirkung der Kombination (I) + (II) die individuellen Effekte der (I) und (II), wenn sie getrennt angewandt werden.

Im Plaque-Reduktionstest zeigt die kombinierte Anwendung der Verbindungen (I) und (II) eine additive Wirksamkeit für jede Konzentration des DD-13 plus $10^{-8}$M/1 Rimantadin und in den Kombinationen des DD-13 (jede Konzentration mit $10^{-9}$M/1 oder $10^{-10}$M/1 Rimantadin) eine klare synergetische Wirksamkeit (seltener eine additive Wirksamkeit), d.h., daß eine günstige Kombination der Wirksamkeiten beider Verbindungen - Summation oder häufiger - Synergismus vorhanden ist.

Bei den Eincyclus- und Mehrcyclentests führte die Kombination der Verbindungen (I) + (II) zu einem deutlichen Verstärken der antivirellen Wirksamkeit beider Verbindungen gegenüber dem Grippe-Virus A/H3N2/Texas/77 und dem Virus FPV/A/H7N7/Weibridge. Bei dem Grippe-Virus B/Lii wird ebenso ein deutliches Verstärken der inhibierenden Wirksamkeit des DD-13 in Anweseheit von Rimantadin festgestellt, bei welchem man weiß, daß der letztere allein angewandt gegen über den Stämmen des Grippe-Virus B nicht aktiv ist (oder seine Wirksamkeit ist unwesentlich).

Es ist klar, daß die Anwendung der Verbindungen (I) und (II) in Kombination die Verwendung von niedrigeren Konzentrationen erlaubt - unter deren wirksamen Konzentrationen oder an der unteren Grenze

der Wirksamkeit bei der Anwendung von (I) und (II) selbständig. Dabei zeigt die Kombination eine ausgeprägte antivirelle Wirksamkeit.

Die Entwicklung einer Resistenz des Virus FPV/Grippe-Virus A/H7N7/ gegenüber dem Rimantadin wird deutlich durch das Vorhandensein des DD-13 gehemmt. Auf diese Weise ist die inhibierende Wirksamkeit des Rimantadins (25 mkg/ml) auf das Virus, das 3 Passagen in Anwesenheit von 100 mkg/ml Rimantadin passiert hat, 0 %, 3 Passagen in Anwesenheit von 100 mkg/ml Rimantadin und 60 mkg/ml DD-13 97,14 %, während die inhibierende Wirkung auf das Ausgangsvirus über 99,23 % ist.

Bei der Verwendung von Rimantadin in Kombination mit DD-13 können diese als Pulver, granuliert oder als Wasserlösungen angewandt werden.

Die Vorteile des erfindungsgemäßen Arzneimittels mit Antivirus-Wirksamkeit (eine Kombination von Rimantadin mit dem Tetrahydropyrimidin-Derivat II) sind folgende:

- es zeigt eine stärkere antivirelle Wirksamkeit;
- es zeichnet sich durch eine höhere Selektivität der Wirksamkeit aus;
- es ist wirksam außer bei einer Grippe-Infektion mit Verursacher Grippe-Virus A und ebenso bei Infektionen mit Verursacher Grippe-Virus B;
- die Möglichkeit zum Entstehen von resistenten Mutanten des Grippe-Virus A gegenüber dem Remantadin wird eingeschränkt.

Anhand der nachstehend als Beispiel angeführten Ausführung wird die Erfindung näher erläutert.

Bei dem Plaque-Reduktionstest bei der Prüfung der Wirksamkeit der Kombination des Remantadins mit dem DD-13 (Tabelle 1) $10^{-8}$M/1 Rimantadin plus $10^{-5}$M/1, 3 x $10^{-5}$M/1 oder $10^{-4}$M/1 DD-13 zeigen eine additive Wirksamkeit.

TABELLE 1

| DD-13 M/1 / Rimantadin M/1 | 0 | $10^{-5}$ | $3x10^{-5}$ | $10^{-4}$ | $3x10^{-4}$ | $10^{-3}$ |
|---|---|---|---|---|---|---|
| 0 | | 2,30 ± 3,67 | 15,23 ± 8,03 | 34,34 ± 19,39 | 85,00 | 99,19 |
| $10^{-10}$ | 3,52 ± 7,05 | 6,63 ± 6,22 add. | 26,08 ± 10,62 syn. | 43,87 ± 14,80 syn. | 94,55 syn. | - |
| $10^{-9}$ | 18,37 ± 14,19 | 26,46 ± 5,70 syn. | 29,87 ± 8,42 add. | 53,28 ± 17,09 syn. | 97,27 syn. | 97,27 add. |
| $10^{-8}$ | 48,00 ± 14,45 | 48,94 ±19,45 add. | 57,33 ±19,51 add. | 61,75 ± 22,83 add. | | 98,18 add. |

add. = additiv

syn. = synergetisch

Bei einer niedrigeren Konzentration des Rimantadins $10^{-9}$ oder $10^{-10}$M/1 wird ein deutlicher Synergismus festgestellt. So ist die selbständige Wirksamkeit d.h. die selbständige inhibierende Wirksamkeit des 3 x $10^{-4}$M/1 DD-13 85 % (eine Verminderung der Plaquebildung). Beim Zufügen von $10^{-10}$M/1 Rimantadin, praktisch eine Konzentration ohne Wirksamkeit, wächst die inhibierende Wirksamkeit auf 94,55, und bei Beifügung von $10^{-9}$M/1 Rimantadin (mit einer selbständigen Wirksamkeit - 18,37 % Inhibition) erlangt die inhibierende Wirksamkeit 97,27 %.

Im Mehrcyclentest zeigt die Prüfung der Wirksamkeit der Kombination gegenüber dem Virus FPV (MOI = 0,5) einen deutlichen Synergismus (Tabelle 2).

TABELLE 2

| DD-13 mkg/ml / Rim. mkg/ml | XA-Titer (inf. Titer in PBE/ml) 0 | 15 | 30 | 60 |
|---|---|---|---|---|
| 0 | 2048 / $1.7x10^7$ | 1024 / $5x10^6$ | 512 / $6,9x10^6$ | 256 / $3,9x10^6$ |
| 1 | 32 / $4,2x10^4$ | 32 / $4,2x10^4$ | 32 / $2,9x10^4$ | 32 / $6x10^4$ |
| 5 | 32 / $4,4x10^4$ | 32 / $6,7x10^4$ | 32 / $6,6x10^4$ | 8 / $3,5x10^3$ |

So z.B. führt die Kombination 60 mkg/ml DD-13 mit 5 mkg/ml Rimantadin zu einer 256-maligen Verminderung der Ausbeute des hämaglutivierenden Virus, während die selbständige Wirksamkeit von 60 mkg/ml DD-13 eine viermalige Verminde rung (Senkung) ist, und von 5 mkg/ml Rimantadin eine 64-malige Senkung. Eine synergistische Wirksamkeit wird ausgerechnet auch beim Einschätzen der Ausbeute an infektiösem Virus in den plaquebildenden Einheiten/ml.

Die Wirksamkeit der Kombination Rimantadin plus DD-13 auf die Fortpflanzung der Grippe-Virus A/H3N2/Texas/77 in einer Zellkultur von Kalbsniere in einem Mehrcyclentest (MOI = 0,05) zeichnet sich ebenso durch eine Verstärken der Wirksamkeit (Tabelle 3) aus.

TABELLE 3

| DD-13 mkg/ml / Rim. mkg/ml | XA-Titer (inf. Titer in log. $EID_{50/ml}$) 0 | 12,5 | 25 | 50 |
|---|---|---|---|---|
| 0 | 512 / 5,7 | 256 / 5,3 | 2 / 5,3 | 2 / 2,7 |
| 0,3 | 2 / 4,5 | 0 / 4,3 | 0 / 4,7 | 0 / 2,7 |
| 1 | 0 / 4,7 | 0 / 4,5 | 0 / 4,5 | 0 / 2,7 |

Die Kombination der Verbindungen (I) und (II) zeigt einen verstärkten Effekt auch auf die Fortpflanzung des Grippe-Virus B/Lii in einer Zellkultur von Kalbsniere bei Prüfung in einem Eincyclustest (MOI = 1) (Tabelle 4).

TABELLE 4

| Rim. mkg/ml \ DD-13 | XA-Titer (inf. Titer in log. $EID_{50}$/ml) 0 | 12,5 | 25 | 50 |
|---|---|---|---|---|
| 0 | 8 / 6,3 | 8 / 4,0 | 4 / 2,5 | 4 / 2,0 |
| 5 | 4 / 6,0 | 4 / 4,7 | 4 / 2,5 | 2 / 2,5 |
| 25 | 4 / 4,7 | 4 / 4,0 | 4 / 2,3 | 2 / 2,5 |

Bei der Prüfung in vivo des Modells der experimentellen Infektionen mit Grippe-Viren A/H3N2) Aichi, A/H1N1) Porto Rico 8 und B/Lii erfolgt ebenso eine deutliche synergetische antivirelle Wirksamkeit der Kombination DD-13 plus Rimantadin.

In Tabelle 1 ist der Effekt bei einer Infektion mit Grippe-Virus A/H3N2/Aichi gezeigt. Die Kombination von dem DD-13 18,7 mg/kg 1/213 von $LD_{50}$ und Rimantadin 10 mg/kg, angewandt peroral bei einer 5-tägigen Behandlung (bei einem 12-stündigen Dosen-Interval) vom Tag der Virus-Inokulation an, zeigt den maximalen protektiven Effekt. Er ist gleich oder größer als die Wirksamkeit beider Komponenten, allein oder in Kombination in zwei mal höherer Dosis eingenommen. Dabei werden hohe Werte des Protektiv-Indexes (80 %) auch bei massiven Virus-Inokulumen (über 50 $LD_{50}$) ausgerechnet.

## Ansprüche

Antivirus-Mittel, dadurch **gekennzeichnet**, daß es N-Methyl-1-adamantanmethylamin-Hydrochlorid

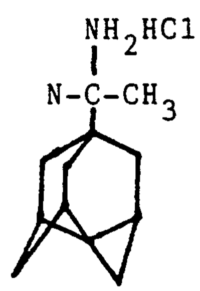

in Kombination mit 1-Morpholinomethyl-tetrahydropyrimidinon

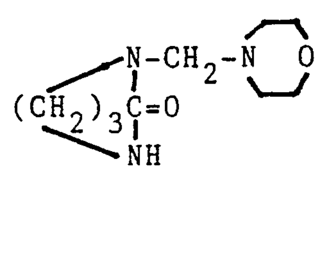

enthält.

Europäisches Patentamt

# EUROPÄISCHER RECHERCHENBERICHT

Nummer der Anmeldung

EP 88 10 0806

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | ARZNEIMITTEL FORSCHUNG, DRUG RESEARCH, Band 35(II), Nr. 8, 1985, Seiten 1269-1275; A. KARPAROV et al.: "Antiviral effect of 1-morpholinomethyl-tetrahydro-2(1H)-pyrimidinone (DD-13) in experimental alphaviral infections in white mice" * Seiten 1274-1275, Absatz 4, "Discussion" * --- | 1 | A 61 K 31/535// (A 61 K 31/535 A 61 K 31:13 ) |
| A | ARZNEIMITTEL FORSCHUNG, DRUG RESEARCH, Band 34 (I), Nr. 1, 1984, Seiten 9-14; A.S. GALABOV et al.: "Antiviral activity of tetrahydro-2(1H)-pyrimidinones and related compounds" * Seiten 13,14, Absatz 4, "Discussion" * ----- | 1 | |
| | | | RECHERCHIERTE SACHGEBIETE (Int. Cl.4) |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 21-09-1988 | BRINKMANN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)